# FASCICULE DE BREVET EUROPEEN

(11) **EP 0 695 142 B1**
(45) Date de publication et mention de la délivrance du brevet: **07.07.1999**
(21) Numéro de dépôt: 94913660.0
(22) Date de dépôt: 18.04.1994
(51) Int. Cl.: A61B 17/064, A61B 17/58, A61B 17/16

(54) **AGRAFE D'OSTEOSYNTHESE ET MATERIEL ANCILLAIRE POUR SA POSE**
OSTEOSYNTHESEKLAMMER UND HILFSINSTRUMENTE ZU IHRER BEFESTIGUNG
OSTEOSYNTHESIS CLIP AND ANCILLARY EQUIPMENT FOR FITTING SAME

(30) Priorité: 19.04.1993 FR 9304635
(43) Date de publication de la demande: 07.02.1996
(73) Titulaire: Savornin, Claude, F-94160 Saint-Mandé (FR)
(72) Inventeur: Savornin, Claude, F-94160 Saint-Mandé (FR)
(74) Mandataire: Dawidowicz, Armand
(86) Numéro de dépôt international: FR9400433
(87) Numéro de publication internationale: WO9423654

(56) Documents cités:
- EP-A- 0 301 898
- WO-A-92/00773
- WO-A-92/17122
- CH-A- 597 838
- FR-A- 2 562 416
- US-A- 2 789 558
- US-A- 4 263 903
- US-A- 4 848 328
- US-A- 5 053 038
- DATABASE WPI Section PQ, Week 8951, Derwent Publications Ltd., London, GB; Class P31, AN 89-376375 & SU,A,1 463 268 (LATV TRAUMA ORTHOPA) 7 Mars 1989 cité dans la demande

## Description

La présente invention concerne une agrafe d'ostéosynthèse destinée à fixer un foyer de fracture ou d'ostéotomie en vue de la consolidation, ainsi qu'un matériel ancillaire pour la pose d'une telle agrafe.

Différentes agrafes d'ostéosynthèse sont connues. On peut citer par exemple l'agrafe décrite dans FR-A-2.668.361, qui est réalisée en un alliage martensitique thermo-élastique à mémoire de forme. Elle comprend deux branches, destinées à être insérées en force de part et d'autre du foyer de la fracture de l'os à réparer, et une base de raccordement. Lesdites branches sont éduquées pour se rapprocher sous l'effet de la température au-dessus de la température de transformation austénitique et la base de raccordement est éduquée pour passer d'une forme rectiligne à une température inférieure à la température de transformation martensitique, à une forme ondulée induisant un raccourcissement de sa longueur, à une température supérieure à la température de transformation austénitique. Une telle agrafe possède des caractéristiques de compression dynamique et d'autorétention. Toutefois elle ne permet pas de régler de façon précise la position des deux fragments osseux à stabiliser car l'opérateur n'est pas maître de la transformation de forme de cette agrafe et ne peut pas faire d'essais avec l'agrafe avant son utilisation, c'est-à-dire avant qu'elle ait sa forme définitive.

On connaît également, par FR-A-2.562.416 (US-A-4.848.328), une agrafe d'ostéosynthèse constituée par une âme à laquelle sont raccordées deux branches latérales. L'âme comprend deux parties inclinées l'une par rapport à l'autre d'un angle obtus ouvert vers l'extérieur. Les branches sont divergentes. Les branches et l'âme ont une section carrée ou rectangulaire. Chacune des branches comporte, sur sa face interne, une succession de saillies en dents de scie formant un dispositif anti-retour. Des ailerons sont prévus sur les faces frontales de chaque branche de l'agrafe. Chacune des branches se termine à son extrémité distale par un biseau délimité par des faces orientées vers l'extérieur dans FR-A-2.562.416 et vers l'intérieur dans US-A-4.848.328. Cette agrafe est destinée essentiellement à fixer un foyer d'ostéotomie en attendant la consolidation de celui-ci, en dessous du plateau tibial. Une telle agrafe, dont les branches sont divergentes, ne permet toutefois pas une bonne compression du foyer d'ostéotomie. Au contraire, il se produit de préférence une distraction, c'est-à-dire un écartement des deux fragments d'os à stabiliser, car la mémoire du métal tend à créer un écartement.

Pour les fractures du scaphoïde carpien, on connaît les broches dites de Kirschner, qui donnent une stabilité médiocre et qui font l'objet d'une migration fréquente, source d'irritation cutanée et de problèmes de tolérance. On connaît également les vis, qui sont difficiles à mettre en place et qui peuvent présenter une prise médiocre dans un pôle proximal scaphoïdien de mauvaise qualité, en particulier dans un foyer de type Schernberg II. On connaît en outre la vis de Herbert, mais elle présente, en plus des inconvénients précités, le défaut majeur de traverser une articulation fondamentale de la première colonne qui est l'articulation scapho trapézienne. Quant aux agrafes du type Warner disponibles sur le marché, elles sont insuffisantes pour ponter une perte de substance importante en matière de pseudoarthrose et elles ne verrouillent pas la rotation des fragments osseux.

On a également proposé (SU-A-1.463.268) une agrafe d'ostéosynthèse constituée d'un élément élastique en forme de U, avec des branches convergentes et un élément de liaison courbé en arc de cercle dont le centre est le point de convergence des branches. L'angle des branches est compris entre 20 et 90°. Ces branches présentent des dents externes. La pose d'une telle agrafe est extrêmement délicate car les branches doivent être écartées élastiquement pour être logées dans des alésages préalablement percés à un angle inférieur à l'angle des branches. La compression fournie est très aléatoire et dépend de la précontrainte fournie par l'écartement préalable des branches. En outre, l'élément de liaison n'est aucunement adapté à la morphologie de l'os fracturé.

La présente invention a pour but de remédier à cet inconvénient en fournissant une agrafe qui réalise une excellente compression du foyer de fracture ou d'ostéotomie, et qui est adaptée au traitement de nombreux types de fractures, de pseudoarthroses ou d'ostéotomie, en particulier au traitement de fractures ou de pseudoarthroses du scaphoïde carpien.

A cet effet, la présente invention a pour objet une agrafe d'ostéosynthèse constituée par une âme et deux branches latérales, l'âme comprenant deux parties inclinées l'une par rapport à l'autre et formant un angle obtus ouvert vers l'extérieur, les deux branches se terminant à leur extrémité distale par un biseau délimité par une face orientée vers l'intérieur et comportant un dispositif anti-retour comprenant des dents, caractérisée en ce que les branches sont convergentes en formant entre elles un angle inférieur à 20° et les dents dudit dispositif anti-retour sont formées sur la face externe desdites branches.

Lors de l'impaction de l'agrafe, les branches qui sont légèrement convergentes ont tendance à s'écarter, mais l'effet de la mémoire du métal tend à les rapprocher, ce qui assure un excellent effet de compression. L'effet anti-retour des dents orientées vers l'extérieur assure le maintien de l'agrafe en compression des fragments osseux de manière bien supérieure à ce qui serait obtenu par des saillies orientées vers l'intérieur, comme le prévoit US-A-4.848.328 constituant l'état de la technique le plus proche, du fait que la qualité de l'os augmente avec l'éloignement par rapport au trait de fracture ou d'ostéotomie.

Dans un mode de réalisation préféré, la section transversale de l'agrafe est circulaire. De cette manière, dans toutes les orientations de mise en place de l'agrafe par rapport à un os, le contact maximum de l'âme avec la surface osseuse est assuré.

Dans un mode de réalisation particulièrement préféré, des griffes internes sont ménagées dans l'angle formé entre l'âme de l'agrafe et chacune des branches latérales, de manière à bloquer la rotation des fragments d'os à stabiliser.

Une agrafe selon l'invention peut être constituée par différents matériaux naturels ou synthétiques appropriés, présentant les propriétés mécaniques correspondant à l'application chirurgicale donnée. Il s'agit par exemple d'un acier à basse teneur en carbone et fusion sous vide, par exemple un alliage 316 LVM, ou d'un acier au titane.

Selon une forme de réalisation préférée de l'invention, l'agrafe est en un matériau biodégradable. L'agrafe selon l'invention est utile pour le maintien en compression de deux fragments d'os, qu'il s'agisse de fracture, de pseudoarthrose ou d'ostéotomie. Elle peut être utilisée par exemple pour le scaphoïde carpien, les phalanges des orteils, les phalanges des doigts, les osselets du carpe, les os du tarse et du pied.

Pour chacune des applications possibles de l'agrafe de la présente invention, l'adaptation se fait simplement en choisissant les angles appropriés et les dimensions pour que l'âme de l'agrafe s'adapte au profil anatomique de l'os concerné.

La mise en place d'une agrafe selon la présente invention s'effectue à l'aide d'un matériel ancillaire simple qui comprend un impacteur préhenseur, tel que décrit par exemple dans WO-A-92 17122, agencé pour recevoir une pluralité de porte-agrafes adaptés aux différentes tailles d'agrafe, le porte-agrafe étant monté de manière amovible sur un support, l'extrémité saillante du porte-agrafe comportant un logement pour l'agrafe. Conformément à l'invention, ce matériel ancillaire comprend en outre un écarteur, une série de viseurs adaptés a la taille des agrafes disponibles, des mèches de forage coopérant avec le viseur pour les points d'implantation et un impacteur définitif.

L'une des applications de l'agrafe de la présente invention est le traitement des fractures ou des pseudoarthroses du scaphoïde carpien. Pour une telle application, il est particulièrement judicieux de choisir, pour l'angle obtus ouvert vers l'extérieur formé par les deux parties de l'âme, un angle de 150°. En outre, les angles formés entre chacune des branches latérales et l'âme sont respectivement de 88° et 58°. Les dimensions des différentes agrafes sont adaptées à la taille du sujet à traiter. Dans le cas du scaphoïde carpien, le site d'implantation de l'agrafe est situé contre le plan ligamentaire inter osseux scapho-grand os et scapho-lunarien, l'implantation se faisant avec une angulation de 30° dans le plan frontal pour permettre de situer les branches de l'agrafe en plein corps scaphoïdien dans la zone où la prise conférera le maximum de solidité sans transfixier les surfaces articulaires adjacentes intercarpiennes et radiocarpiennes.

La présente invention est illustrée plus en détail par référence aux dessins annexés représentant une forme d'exécution particulière, donnée à titre d'exemple non limitatif.
La figure 1 est une vue en élévation d'une agrafe selon la présente invention.
La figure 2 est une vue d'une agrafe selon l'invention, insérée dans un scaphoïde carpien.
La figure 3 est une vue en élévation latérale d'un écarteur.
La figure 4 est une vue en plan d'une extrémité de l'écarteur de la figure 3.
La figure 5 est une vue en coupe d'un viseur.
La figure 6 est une vue de bout du viseur de la figure 5.
La figure 7 est une vue en coupe d'un impacteur préhenseur d'agrafe.
La figure 8 est une vue en élévation latérale d'un impacteur définitif.
La figure 9 est une vue en élévation latérale de l'impacteur de la figure 8, à 90° de la vue de la figure 8.

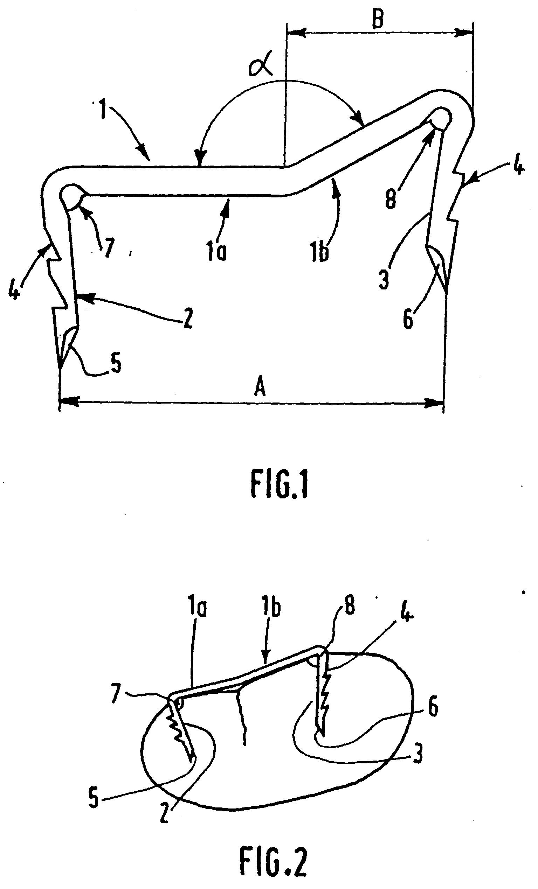

L'agrafe représentée sur la figure 1 est constituée par une âme 1 comportant deux parties 1a et 1b, et par deux branches 2 et 3. Les deux parties 1a et 1b de l'âme forment un angle α obtus ouvert vers l'extérieur. Chacune des branches 2 et 3 forme, avec l'âme 1, un angle aigu tel que les deux branches soient légèrement convergentes l'angle entre les branches étant inférieur à 20°. Dans le cas particulier où l'agrafe est destinée à être utilisée pour le scaphoïde carpien, l'angle α est de préférence de 150°, l'angle formé entre la branche 2 et la partie la est de préférence de 88°, et l'angle formé entre la branche 3 et la partie 1b est de préférence de 58°. Cette configuration particulière permet une adaptation optimale de l'âme de l'agrafe sur l'os et confère aux branches leur caractère faiblement convergent.

Chaque branche comprend, sur sa face extérieure, des dents 4. Chaque branche peut comporter, par exemple de 2 à 4 dents, de préférence 4. Les dents 4 peuvent être formées, comme dans l'exemple représenté, par usinage des branches. Cependant, de préférence, les dents sont formées en saillie par rapport à l'ensemble de la branche, par exemple par formage à froid ou toute autre technique convenable.

Les branches 2 et 3 présentent respectivement à leur extrémité distale, un biseau 5, 6 dont la face est orientée vers l'intérieur.

L'âme et les branches présentent une section circulaire. Ainsi, quelle que soit l'orientation de l'agrafe par rapport à l'os, le contact entre l'âme et l'os sera optimal, alors que si l'âme présente une section rectangulaire ou carrée, le contact sera plus ou moins important suivant l'orientation.

Une griffe 7 est ménagée dans l'angle formé par la branche 2 et la partie la de l'âme, et une griffe 8 dans l'angle formé entre la branche 3 et la partie 1b de l'âme. Ces griffes ont pour résultat d'empêcher la rotation de l'un des fragments d'os par rapport à l'autre.

Une agrafe selon l'invention présentant les caractéristiques reproduites sur la figure 1 est parfaitement adaptée pour être utilisée sur le scaphoïde carpien. Les dimensions des branches seront adaptées à la taille du sujet à traiter. Par exemple, la longueur des branches 2 et 3 peut être de 8 mm ou 10 mm constante ou variable selon les tailles d'agrafes, l'écartement A entre les extrémités distales des branches peut être compris entre 12 et 18 mm, la longueur B de la projection orthogonale de la partie 1b sur la droite portant la partie 1a entre 8 et 9mm. Le diamètre de la section circulaire de l'âme et des branches est de 1 à 1,20 mm. Une gamme de 7 tailles différentes à l'intérieur de ces fourchettes permet de couvrir l'essentiel des besoins. Le choix particulier des angles et des dimensions de l'agrafe permet une adaptation très précise de l'agrafe à la forme de l'os, ce qui rend l'ablation de l'agrafe non nécessaire lorsque l'os est consolidé. En outre, cette agrafe n'a aucun retentissement sur les amplitudes du poignet, du fait qu'elle ne crée aucun effet butoir au niveau intra articulaire radiocarpien.

L'invention concerne également le matériel ancillaire pour la pose de l'agrafe.

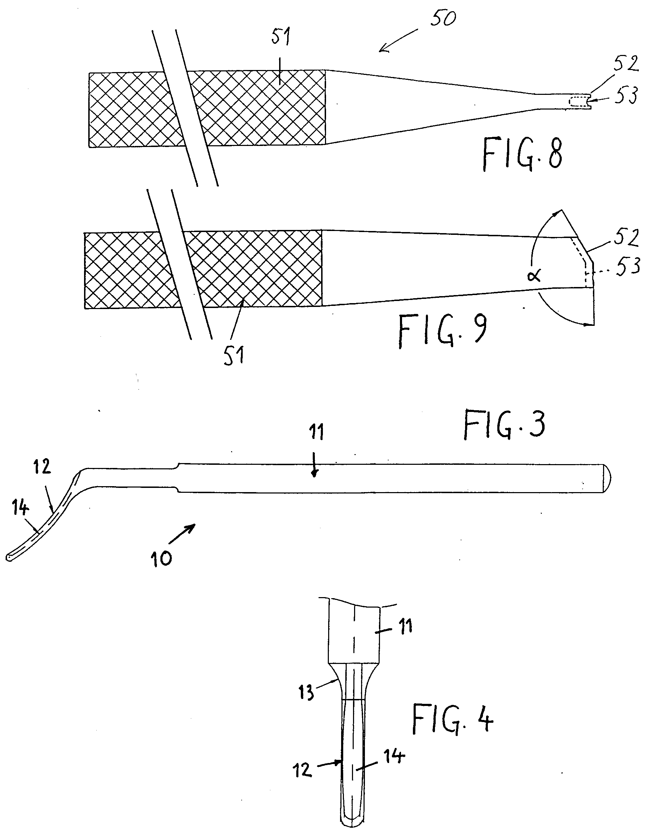

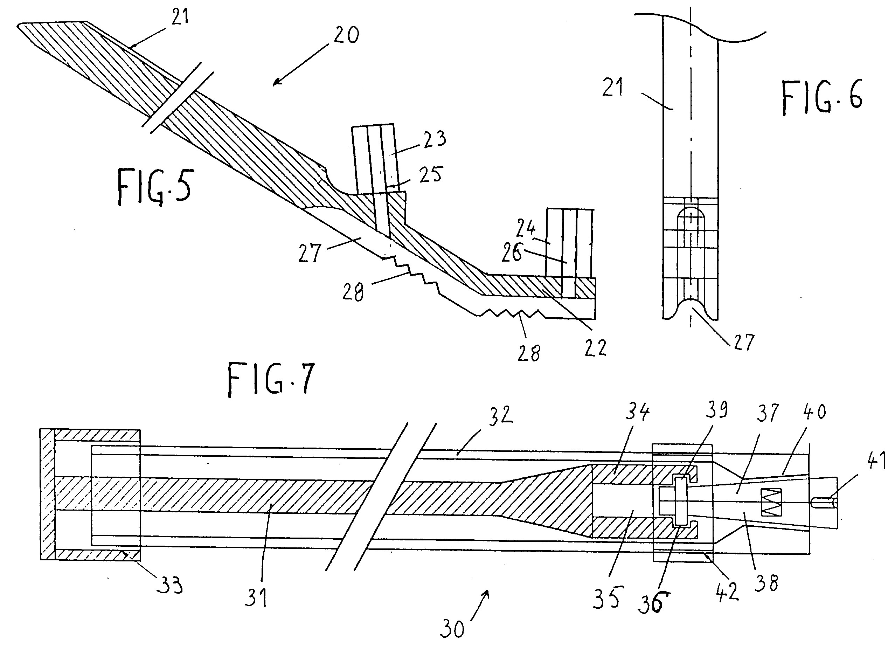

Ce matériel ancillaire comprend un écarteur 10 (figures 3 et 4) destiné, dans le cas d'une fracture du scaphoïde carpien, à être introduit entre le scaphoïde et le radius de manière à produire un rapprochement des deux segments osseux. L'écarteur 10 comprend un manche 11 terminé par une cuiller concave 12 raccordée par un congé 13. La cuiller 12 comporte une gorge longitudinale 14.

Le chirurgien choisit ensuite, en fonction des dimensions de l'os, un viseur 20 (figures 5 et 6). Le viseur comprend un manche 21 terminé par une extrémité 22 inclinée par rapport au manche 21 d'un angle égal à l'angle α de l'agrafe 1. Le viseur 20 comporte, de part et d'autre de la pliure d'extrémité, deux saillies annulaires supérieures 23 et 24 dont les alésages 25 et 26 respectivement débouchent dans une creusure semi-circulaire inférieure 27. L'inclinaison et l'écartement des alésages 25 et 26 correspondent, en combinaison avec la forme de la face inférieure du viseur, aux dimensions de l'agrafe à poser. La face inférieure du viseur comporte en outre des dents 28 empêchant le glissement sur l'os.

Le chirurgien, après mise en place du viseur, perce un premier trou de fixation au moyen d'une mêche introduite dans l'alésage 25. Il utilise ensuite une seconde mêche pour percer le second trou de fixation. Il enlève ensuite les mèches. L'os est prêt à recevoir l'agrafe.

L'agrafe est alors mise en place sur un impacteur préhenseur 30 représenté à la figure 7. Celui-ci est constitué d'un support 31 coulissant dans un tube 32. Le support 31 comprend une queue taraudée 33 vissée sur un filetage extérieur du tube 32 et une tête 34 munie d'une creusure cylindrique axiale 35 comportant une gorge périphérique interne 36. Un porte-agrafe adapté à l'agrafe choisie et constitué de deux demi-coquilles 37, 38 est monté dans la creusure 35 par un bourrelet périphérique 39. Les demi-coquilles 37, 38, sont tronconiques après le bourrelet 39 et sont logées dans une extrémité interne tronconique 40 du tube 32 dont elles font saillie extérieurement. Le serrage et le desserage se font par dévissage et vissage de la queue 33. L'extrémité saillante porte un logement d'agrafe 41. Une bague taraudée 42 de démontage est montée sur le tube 32. L'impacteur préhenseur 30 permet la mise en place précise et l'enfoncement de l'agrafe.

L'enfoncement complet est assuré au moyen d'un impacteur définitif 50 représenté aux figures 8 et 9. Celui-ci comporte un manche moleté 51 et une tête 52 munie d'une gorge 53 et profilée à la forme de l'agrafe.

## Revendications

1. Agrafe d'ostéosynthèse constituée par une âme (1) et deux branches latérales (2, 3), l'âme (1) comprenant deux parties (1a, 1b) inclinées l'une par rapport à l'autre et formant un angle obtus (α) ouvert vers l'extérieur, les deux branches (2, 3) se terminant à leur extrémité distale par un biseau (5, 6) délimité par une face orientée vers l'intérieur et comportant un dispositif anti-retour comprenant des dents (4),
caractérisée en ce que les branches (2, 3) sont convergentes en formant entre elles un angle inférieur à 20° et les dents (4) dudit dispositif anti-retour sont formées sur la face externe de chacune des branches (2, 3).

2. Agrafe selon la revendication 1,
caractérisée en ce que la section transversale de l'âme (1) et des branches (2, 3) de l'agrafe (1) est circulaire.

3. Agrafe selon l'une des revendications 1 et 2,
caractérisée en ce qu'une griffe interne (7, 8) est ménagée dans l'angle formé entre l'âme (1) de l'agrafe et chacune des branches latérales (2, 3).

4. Agrafe selon l'une des revendications 1 à 3,
caractérisée en ce qu'elle est constituée par un acier à basse teneur en carbone et fusion sous vide.

5. Agrafe selon l'une des revendications 1 à 3,
caractérisée en ce qu'elle est constituée par un acier au titane.

6. Agrafe selon l'une des revendications 1 à 3,
caractérisée en ce qu'elle est en un matériau biodégradable.

7. Agrafe selon l'une des revendications 1 à 6,
caractérisée en ce que l'angle obtus ouvert vers l'extérieur formé par les deux parties (1a, 1b) de l'âme (1) est un angle de 150° et que les angles formés entre chacune des branches latérales (2, 3) et l'âme (1a, 1b) sont respectivement de 88° et 58°.

8. Agrafe selon l'une des revendications 1 à 7,
caractérisée en ce que la longueur des branches (2, 3) est fonction de la taille de l'agrafe.

9. Agrafe selon l'une des revendications 1 à 7,
caractérisée en ce que la longueur des branches (2, 3) est constante pour toutes les tailles d'agrafe.

10. Matériel ancillaire comprenant un impacteur préhenseur (30) agencé pour recevoir une pluralité de porte-agrafes (37, 38) adaptés aux différentes tailles d'agrafes, le porte-agrafe (37, 38) étant monté de manière amovible sur un support (31), l'extrémité saillante du porte-agrafe (37, 38) comportant un logement (41) pour l'agrafe, pour la pose d'agrafes de tailles différentes selon l'une des revendications 1 à 9,
caractérisé en ce qu'il comprend :
- un écarteur (10), comprenant un manche (11) comprenant une cuiller concave (12) raccordée par un congé (13), ladite cuiller (12) comportant une gorge longitudinale (14),
- une pluralité de viseurs (20) adaptés aux différentes tailles d'agrafes et comportant des alésages (25, 26) de guidage de mèches de perçage dont l'inclinaison et l'écartement correspondent, en combinaison avec la forme de la face inférieure du viseur, aux dimensions de l'agrafe à poser et,
- un impacteur définitif (50) muni d'une tête (52) profilée à la forme des agrafes et comportant une gorge (53),
- ledit support (31) du porte-agrafe (37, 38) étant coulissant dans un tube (32).

## Claims

1. Osteosynthesis clip formed by a core (1) and two lateral arms (2, 3), the core (1) comprising two portions (1a, 1b) which are inclined relative to each other and form an outwardly open obtuse angle (α), the two arms (2, 3) terminating at their distal end in a bevel (5, 6) defined by an inwardly orientated face and comprising a non-return device having teeth (4), characterised in that the arms (2, 3) are convergent, forming an angle of less than 20° therebetween, and the teeth (4) of said non-return device are formed on the external face of each of the arms (2, 3).

2. Clip according to claim 1, characterised in that the cross-section of the core (1) and the arms (2, 3) of the clip (1) is circular.

3. Clip according to one of claims 1 and 2, characterised in that an internal claw (7, 8) is disposed in the angle formed between the core (1) of the clip and each of the lateral arms (2, 3).

4. Clip according to one of claims 1 to 3, characterised in that it is formed from steel with a low carbon content and vacuum-fused.

5. Clip according to one of claims 1 to 3, characterised in that it is formed from titanic steel.

6. Clip according to one of claims 1 to 3, characterised in that it is formed from a biodegradable material.

7. Clip according to one of claims 1 to 6, characterised in that the outwardly open obtuse angle formed by the two portions (1a, 1b) of the core (1) is an angle of 150°, and in that the angles formed between each of the lateral arms (2, 3) and the core (1a, 1b) are 88° and 58° respectively.

8. Clip according to one of claims 1 to 7, characterised in that the length of the arms (2, 3) is dependent on the size of the clip.

9. Clip according to one of claims 1 to 7, characterised in that the length of the arms (2, 3) is constant for all clip sizes.

10. Ancillary equipment, comprising a prehensory impactor (30) designed to accommodate a plurality of clip-carriers (37, 38) adapted to different clip sizes, the clip-carrier (37, 38) being detachably mounted on a support (31), the projecting end of the clip-carrier (37, 38) comprising a housing (41) for the clip, for fitting clips of different sizes according to one of claims 1 to 9, characterised in that it comprises:
- a spacer (10), comprising a handle (11) comprising a concave spoon (12) attached by a bead (13), said spoon (12) comprising a longitudinal groove (14),
- a plurality of sights (20), adapted to the different clip sizes and comprising guidance bores (25, 26) for drill bits, the inclination and spacing of which correspond, in combination with the shape of the lower face of the sight, to the dimensions of the clip to be fitted, and
- a definitive impactor (50), provided with a head (52) profiled to the shape of the clips and having a groove (53),
- said support (31) for the clip-carrier (37, 38) being slidable in a tube (32).

## Patentansprüche

1. Osteosyntheseklammer, die gebildet ist durch einen Steg (1) und zwei seitliche Arme (2, 3), wobei der Steg (1) zwei Teile (1a, 1b) aufweist, die relativ zueinander geneigt sind und einen stumpfen Winkel (α) bilden, der sich nach außen öffnet, wobei die zwei Arme (2, 3) an ihrem distalen Ende mit einer Abschrägung (5, 6) enden, die durch eine Fläche begrenzt ist, die zum Inneren gerichtet ist, und eine Sicherungsvorrichtung gegen Rückbewegung aufweisen, die Zähne (4) umfaßt,
**dadurch gekennzeichnet,** daß die Arme (2, 3) konvergierend sind, indem sie miteinander einen Winkel bilden, der kleiner als 20° ist, und daß die Zähne (4) der genannten Sicherungsvorrichtung gegen Rückbewegung auf der Außenfläche jedes der Arme (2, 3) gebildet sind.

2. Klammer nach Anspruch 1, **dadurch gekennzeichnet,** daß der Transversalquerschnitt des Steges (1) und der Arme (2, 3) der Klammer (1) kreisförmig ist.

3. Klammer nach einem der Ansprüche 1 und 2, **dadurch gekennzeichnet,** daß eine innere Klaue (7, 8) in dem Winkel gebildet ist, der zwischen dem Steg (1) der Klammer und jedem der seitlichen Arme (2, 3) gebildet ist.

4. Klammer nach einem der Ansprüche 1 bis 3, **dadurch gekennzeichnet,** daß sie aus einem Stahl besteht, der einen niedrigen Gehalt an Kohlenstoff aufweist und unter Vakuum geschmolzen ist.

5. Klammer nach einem der Ansprüche 1 bis 3, **dadurch gekennzeichnet,** daß sie aus einem Stahl mit Titan besteht.

6. Klammer nach einem der Ansprüche 1 bis 3, **dadurch gekennzeichnet,** daß sie aus einem biologisch abbaubaren Material besteht.

7. Klammer nach einem der Ansprüche 1 bis 6, **dadurch gekennzeichnet,** daß der stumpfe Winkel, der sich nach außen öffnet und durch die zwei Teile (1a, 1b) des Steges (1) gebildet ist, ein Winkel von 150° ist und daß die Winkel, die zwischen jedem der seitlichen Arme (2, 3) und dem Steg (1a, 1b) gebildet sind, 88° bzw. 58° betragen.

8. Klammer nach einem der Ansprüche 1 bis 7, **dadurch gekennzeichnet,** daß die Länge der Arme (2, 3) von den Abmessungen der Klammer abhängig ist.

9. Klammer nach einem der Ansprüche 1 bis 7, **dadurch gekennzeichnet,** daß die Länge der Arme (2, 3) für sämtliche Abmessungen der Klammer konstant ist.

10. Hilfsinstrument, mit einem Eintreiber / Greifer (30), der darauf eingerichtet ist, eine Mehrzahl von Klammerträgern (37, 38) aufzunehmen, die an unterschiedliche Abmessungen von Klammern angepaßt sind, wobei der Klammerträger (37, 38) in lösbarer Weise an einer Halterung (31) angeordnet ist, wobei das vorstehende Ende des Klammerträgers (37, 38) eine Aufnahme (41) für die Klammer aufweist, wobei das Hilfsinstrument zur Befestigung von Klammern unterschiedlicher Abmessungen nach einem der Ansprüche 1 bis 9 vorgesehen ist,
**dadurch gekennzeichnet,** daß es aufweist,
- ein Zwischenstück (10), das einen Griff (11) aufweist, der einen konkaven Löffel (12) aufweist, an den sich eine Kehle (13) anschließt, wobei der genannte Löffel (12) eine in Längsrichtung verlaufende Vertiefung (14) aufweist,
- eine Mehrzahl von Visieren (20), die an unterschiedliche Größen von Klammern angepaßt sind und Bohrungen (25, 26) zur Führung von Bohrern aufweisen, wobei die Neigung und der Abstand der Bohrungen (25, 26) in Kombination mit der Form der unteren Fläche des Visiers den Dimensionen der zu befestigenden Klammer entsprechen und
- einen endgültigen Eintreiber (50), der mit einem Kopf (52) versehen ist, der an die Form der Klammern profiliert ist und eine Vertiefung (53) aufweist,
- wobei die Halterung (31) des Klammerträgers (37, 38) verschiebbar in einem Rohr (32) angeordnet ist.
